# EUROPEAN PATENT SPECIFICATION

(11) **EP 1 677 594 B1**
(45) Date of publication and mention of the grant of the patent: **12.01.2011**
(21) Application number: 04770599.1
(22) Date of filing: 10.10.2004
(51) Int. Cl.: A01N 1/02

(54) **METHOD FOR FREEZING, THAWING AND TRANSPLANTATION OF VIABLE CARTILAGE**
VERFAHREN ZUM TIEFGEFRIEREN, AUFTAUEN UND TRANSPLANTIEREN VON LEBENSFÄHIGEM KNORPEL
PROCEDE DE CONGELATION, DE DECONGELATION ET DE TRANSPLANTATION D'UN CARTILAGE VIABLE

(30) Priority: 09.10.2003 US 509546 P; 15.01.2004 US 536508 P
(43) Date of publication of application: 12.07.2006
(73) Proprietor: Core Dynamics Limited, Hamilton HM EX (BM)
(72) Inventor: DAMARI, Udi, 55900 Ganiey Tikva (IL); LEVI HOLTZMAN, Rivi, 76466 Rehovot (IL); RZEPAKOVSKY, Victor, 74062 Ness Zionna (IL)
(74) Representative: Minoja, Fabrizio
(86) International application number: PCT/IL2004/000929
(87) International publication number: WO 2005/032251

(56) References cited:
- WO-A-02/32225
- WO-A-91/06213
- WO-A-98/10231
- WO-A-03/056919
- US-B1- 6 488 033
- K. MULDREW ET AL.: "Localization of freezing injury in articular cartilage." CRYOBIOLOGY, vol. 31, 1994, pages 31-38, XP002313331
- K. MULDREW & ET AL.: "Transplantation of articular cartilage following a step-cooling cryopreservation protocol" CRYOBIOLOGY, vol. 43, 2001, pages 260-267, XP002313330 cited in the application
- L. CSÖNGE ET AL.: "Banking of osteochondral allografts, part II. Preservation of chondrocyte viability during long-term storage." CELL AND TISSUE BANKING, vol. 3, 2002, pages 161-168, XP002313332
- STEENSEL VAN M A M ET AL: "OPTIMIZATION OF CRYOPRESERVATIVE PRODEDURES FOR HUMAN ARTICULAR CARTILAGE CHONDROCYTES" ARCHIVES OF ORTHOPAEDIC AND TRAUMA SURGERY, SPRINGER VERLAG, DE, vol. 113, no. 6, 1994, pages 313-321, XP000925592 ISSN: 0936-8051 cited in the application

## Description

### FIELD OF THE INVENTION

The present invention relates to freezing, thawing and transplantation of viable cartilage.

### LIST OF REFERENCES

The following references are brought to facilitate description of the background of the present invention, and should not be construed as limiting the patentability of the invention:
1. Muldrew, K. et al., Cryobiology 43, 260-267 (2001);
2. Higgs, G.B. and Boland, A.L, Proceedings of the International cartilage Repair Society's Second Symposium, November 1998;
3. McGoveran, B.M. et al., The Journal of Knee Surgery, vol.15, No.2 Spring 2002;
4. Williams, SK. et al, The Journal of Bone and Joint Surgery (American), 85:2111-2120 (2003).
5. US patent 5,131,850 to Kelvin G. M.;
6. US patent 6,488,033 to Cerundolo D. G;
7. US patent 5,873,254 to Arav A.;
8. US patent application 20030064357 to Arav A.;
9. PCT/IL03/00026 to Arav A.
10. US patent 6,740,484 to Khirabadi et al.

The above publications will be referenced below by indicating their number from the above list.

### BACKGROUND OF THE INVENTION

Adult cartilage is a connective tissue populated by chondrocytes embedded in a dense extra cellular matrix (ECM) composed of a collagenous fiber network. Functional articular cartilage is critical to proper joint function. Unfortunately articular cartilage has low self-repair ability and therefore defects are prone to cause abnormal joint biomechanics, leading in the long run to degenerative changes.

Treatments strive for a pain-free range of motion in the joint that provides enduring function, which enable a young patient to participate in a wide range of activities [3]. The list of operative treatment options for hyaline cartilage injury currently utilized by orthopaedic surgeons include microfracture, chondral abraisonplasty, osteochondral autograft transplantation (OATS or mosaicplasty), autologous chodrocyte transplantation (ACI), fresh osteochondral allografts, autologous perichondral or periosteal transplantation, and transplantation of bioabsorbable or non-bioabsorbable matrices. The ultimate alternative is, of course, partial or total replacement of the joint with prosthesis [2]. Nevertheless the management of large full thickness osteochondral lesions remains an enigma.

Currently, the method of choice to repair cartilage damage that is greater than 3 cm in diameter and 1 cm in depth is the implantation of tissue [3]. Normally, the implanted tissue (comprising bone and cartilage) is taken from a cadaver, from a site that is most similar to the organ that is in need of repair in the recipient. This allows the implanted tissue to have the most similar shape, arrangement (e.g. of bone and cartilage tissue) and weight bearing characteristics as the tissue of the implant site. This implant (or graft) is often named "*allograft*" since the graft is taken from one individual and implanted in another.

Currently, cartilage transplantation is limited to fresh grafts. This is mainly due to the fact that the post-implantation viability of the chondrocytes is necessary for the long-term maintenance of the biomechanical properties of the cartilage graft. Chondrocytes in cartilage are enclosed in lacunas within the ECM, such that if a cell dies within a lacuna it cannot be replaced by a cell migrating thereto. Thus, unlike bone grafts that may comprise dead bone tissue (which will be later populated by bone cells that would migrate into the implant) the cartilage graft must provide viable cartilage cells.

However, cartilage cells can be maintained viable within cartilage for a restricted period of time and must therefore be transplanted within a few days (no more than 14) from the moment of donation [4, 5]. This does not normally allow sufficient time to test the donated tissue for undesired agents or traits such as transmittable diseases. It also reduces the chances of finding the best donor-recipient match.

Long-term banking can ensure an adequate and safe supply of viable cartilage and the need for matching tissue type and site-specific requirements (size, cartilage thickness etc.).

Isolated chondrocytes (i.e. not within cartilage ECM) have been successfully cryopreserved, [1] but so far cartilage cryopreservation and control rates of freezing and thawing did not achieve an acceptable degree of cartilage viability [3].

US Patent No. 5,131,850 [4] purported to disclose in 1992 a method for cryopreserving musculoskeletal tissues such as cartilage. However, it is commonly accepted that this cannot be achieved and the same assignee clearly accepts (in a later patent) that "*Osteoarticular allografts, however, have not typically been used because osteoarticular cartilage cells do not survive the freezing or cryopreservation process*" [6].

In the work done by Muldrew et al. [3] cartilage was cryopreserved by exposure of the cartilage to step-wise decreasing external temperature. This method resulted in improved chondrocytes recovery within the thawed cartilage. Nevertheless there was considerable variability in the cells' survival rates within the experimental group and the mean cell recovery was not appreciably improved.

US patent 6,740,484 [10] discloses a method for vitrifying tissue (including cartilage segments) as a method of overcoming the problems associated with freezing. Vitrification means solidification, as in glass, without ice crystal formation. This is normally done by raising the glass transition temperature and reducing the homogenous nucleation temperature, which is normally done by adding cryoprotectants at high concentrations. One of the drawbacks of this method is that the added cryoprotectants may be hazardous and/or toxic and must be removed (or at least significantly diluted) upon melting.

US patent 6,740,484 [10] also discloses a method for melting tissue (including cartilage). In this method the vitrified material is first warmed at a relatively slow rate (generally below 50°C/minute) to a temperature between -80°C and the glass transition temperature. Then the material is warmed at a faster warming rate (generally above 80°C/minute) to a temperature above -75°C, at which temperature the solution is sufficiently fluid that the tissue may be removed therefrom. This rapid rate is achieved by changing the temperature of the environment in which the receptacle containing the solution is placed. Then the cryoprotectants are removed.

An advanced technology for freezing is the "Multi-temperature gradient" (MTG) directional solidification, which is based on the invention disclosed in US 5,873,254. In this technology, the sample is moved at a constant velocity (V) through temperature gradients (G) so the cooling rate (G x V), ice front propagation are controlled and the velocity of the movement of the sample determines the morphology of the ice crystals formed within the sample. This method also enables the incorporation of controlled seeding into the freezing process. This method was utilized for example for freezing various biological samples in containers of various dimensions (e.g. sperm, blood, oocytes 7, 8) and even ovaries [9].

### Glossary

The term "*viable cartilage*" in the context of this invention means any tissue comprising viable cartilage cells (chondrocytes) embedded in cartilage extra cellular matrix (ECM), whether or not comprising other elements including cells of other types and/or ECM. Such cartilage may be taken from any source, including, for example, hyaline cartilage (such as the articular cartilage present in the tip of joints, such as hip, knee, shoulder, elbow, etc.) and fibrocartilage (such as the cartilage present in the ears and in the inner parts of the nose). It may be for example menisci or an osteochondral tissue (i.e. tissue comprising both cartilage and bone). Osteochondral tissue is often harvested or grafted in the form of an osteochondral plug or osteochondral cylinder. Non limiting examples for non-cartilage cells and tissues that may be included in a viable cartilage sample are cells and/or extra cellular matrix of bone, tendon, ligament, etc. In order for the cartilage to be deemed viable, at least some of the chondrocytes embedded therein must be viable, preferably 30% or more, 50% or more, 65% or more or even 69% or more (e.g. using the live/dead ratio assay detailed below).

The term "*viable [cells*/*tissue]*" in the context of this invention means (as the context requires) cells or tissue comprising cells that are capable of surviving provided that they are given the necessary conditions (e.g. nutrients, temperature and the like). When applied to frozen cells/tissue, the term "*viable*" denotes such cells or tissues that are capable of remaining viable after being thawed.

The term "*frozen*", in the context of this invention, means being or becoming a solid that comprises ice crystals at a temperature in which all biological processes are practically ceased.

The term "*freezing temperature*" in the context of this invention means a temperature below which a liquid to crystal phase transition occurs.

The term "*melting temperature*" in the context of this invention means a temperature above which there is no more recrystalization of ice but only transition from ice to liquid. Normally for solutions used for freezing rather than vitrification, the melting temperature is about -10 to -5°C.

The term "*glass transition temperature*" in the context of this invention means a temperature which below it a system is increased in its viscosity until it is no longer liquid but is turned into an amorphous solid in a stable thermodynamic state. Normally for solutions used for freezing rather than vitrification, the glass transition temperature is about -120 to - 110°C.

The above temperatures are different for each solution, and are a product of the solution's composition. The temperatures can be determined, at least approximately, by methods known in the art. In the context of this invention, a temperature is deemed to be "*about*" or "*substantially equal*" to a given temperature (e.g. to a melting temperature) when the difference in temperature is no greater than 10°C, more preferably no more than 5°C, 1°C or even 0.5°C, and most preferably no more than 0.01°C.

The term "*receptacle*" in the context of this invention means any sort of container capable of containing viable cartilage and capable of withstanding the freezing and/or thawing of the invention, such that the viable cartilage may be protected from spillage and/or contamination. Non-limiting examples of such receptacles are tubes, bags, straws, sacs, vials and laboratory dishes.

The term "*cryopreservation solution*" refers to any solution or media in which biological material (such as cartilage) is immersed before cryopreservation. Typically, cryopreservation solutions contain a balanced salt solution such as phosphate buffered saline and at least one cryoprotectant. Cryoprotectants are substances that reduce the damage incurred by the cells or tissues during freezing and/or thawing. Most freezing solutions are composed of intracellular cryoprotectants (e.g. DMSO, glycerol, ethylene glycol, polyethylene glycol, 1,2-propanediol, formamide) and/or extra cellular cryoprotectants (Sugars, proteins, carbohydrates such as: Hydroxy Ethyl Starch, dextran, etc.). Some optional cryopreservation solutions do not comprise glycosaminoglycans. Preferred cryopreservation solutions for freezing are such that comprise significantly less cryoprotectants or a significantly lower concentration of cryoprotectants than are conventionally used for vitrification. Accordingly, a typical vitrification solution would comprise at least 30-40% v/v of permeating cryoprotectants (e.g. DMSO, glycerol, ethylene glycol, polyethylene glycol, 1,2-propanediol and/or formamide) while a typical freezing solution would have even 10-15% v/v or less.

### SUMMARY OF THE INVENTION

The present invention is aimed at providing a patient having impaired cartilage in an organ, with corresponding viable cartilage. This method may include freezing and/or thawing of viable cartilage, as necessary.

Accordingly, by one aspect the present invention provides a method of providing a patient having impaired cartilage in an organ at a target site, with corresponding viable cartilage, the method comprising:
(a) providing a receptacle containing viable cartilage having shape and size compatible with the target site in the organ in a cryopreservation solution at a temperature above the freezing temperature of the cryopreservation solution;
**(b)** directionally cooling said viable cartilage to a temperature below said freezing temperature at a cooling rate of 0.01°C/min to 3°C/min, whereby the receptacle is moved along one or more consecutive temperature gradients ranging from a temperature above said freezing temperature to a temperature below said freezing temperature, thereby generating frozen corresponding viable cartilage.

In addition, the method may comprise after step (b):
**(c)** transferring the receptacle to storage at a temperature below the freezing point of said cryopreservation solution.

Optionally, the cooling of above Step (b) may be between 0.1°C/min to 3°C/min.

The velocity of movement of the method of the invention may be any velocity that would achieve the desired cooling rate, and preferably between 0.002 mm/sec and 5 mm/sec. The temperature gradients along which the sample is moved may be any gradients that would achieve the desired cooling rate, and preferably at least one of them should be between 0. 1°C/mm to 50°C/mm.

In addition, the method of freezing detailed above may also comprise seeding of freezing as well known in the art (e.g. patent 5,873,254). Such seeding may be performed by contacting the receptacle during the freezing process with a cold substance, having a temperature below the freezing point of the solution. Examples for such substances are any cold solid object (e.g. metal) or a cold fluid (e.g. liquid nitrogen or its vapor) or a cloth or wool soaked in a cold liquid (e.g. nitrogen). This may be performed at any time that is prior to the initiation of spontaneous freezing within the receptacle

According to yet another aspect of the present invention, a method is provided for thawing frozen viable cartilage that was frozen in a solution, said method comprising:
**(i)** providing a receptacle containing frozen viable cartilage at an initial temperature below the glass transition temperature of the solution;
**(ii)** warming said viable cartilage from said initial temperature to an intermediate temperature being at least about said glass transition temperature or above said glass transition temperature but no more than the temperature wherein recrystalization would begin to occur at any point in the cartilage;
**(iii)** warming said viable cartilage from said intermediate temperature to a temperature that is at least substantially equal to the melting temperature of the solution, by removing said viable articular cartilage tissue from said receptacle and contacting the said viable articular cartilage tissue with an environment having a temperature of 0°C or more; warming being at a rate sufficiently high to minimize recrystalization; thereby obtaining thawed viable articular cartilage, wherein said viable articular cartilage tissue is connected to a pulling member and the removing of said viable articular cartilage tissue from said receptacle is executed by pulling on said pulling member.

The warming rate of step (ii) may be the same as, or different than, that of step (iii). In fact, the warming of step (ii) may be executed at a slow rate so as to minimize the chances of fracture of said viable cartilage. Optionally, each of said steps (ii) and/or (iii) may comprise more than one warming rate.

Since it is difficult to ascertain the exact temperature at which recrystalization would begin to occur at some point in the cartilage (at a location closer to the heat source) it is preferred that step (iii) would begin when the temperature of the cartilage at its warmest point (e.g. close to a heat source) is significantly lower than -10°C, such as -20 to -80°C, or even -40 to -80°C or -50 to - 70°C.

A preferred rate of warming in above step (ii) is between 0.1°C/min and 200°C/min, and more preferably 90°C/mm. Likewise, a preferred rate of warming in above step (iii) is between 50°C/min and 1000°C/min, or 100°C/min and 1000°C/min, or 200°C/min.

Preferably the temperature of the environment in step (ii) would be even higher, such as 22°C or more, 37°C or more, or even 50°C or 70°C or more. The above steps are expected to be capable of increasing the rate of warming (and thus reducing the period when recrystalization may occur) since once the receptacle is removed, heat exchange may occur directly between the frozen cartilage and the solution in which it was frozen, without the receptacle being a potentially insulating barrier, without spending energy to warm the receptacle itself.

The removal of the frozen cartilage is done by pulling it out of the receptacle at a temperature sufficiently high to allow separation of the frozen cartilage from the receptacle without significantly damaging the structure of the receptacle and/or tissue, e.g. ca. -20°C.

In a specific embodiment a screw connected to a pulling member (e.g. thread or wire) may be screwed into the bone segment of an osteochondral cylinder before freezing, such that the bone would freeze around the screw. The step of removing the frozen cartilage from the receptacle in such case is executed by pulling on the pulling member, preferably without damaging the receptacle.

In cases where the cartilage should remain sterile, care should be taken that the cartilage be maintained in a sterile environment once the receptacle is opened. For example, above steps (i) and (ii) may be executed under a sterile hood and the environment with which the frozen cartilage is contacted must be sterile as well (e.g. a warm sterile solution).

The provided cartilage may be given the desired shape and/or size either before or after freezing. For example, cylinders of many shapes and sizes may be frozen and stored for use upon need. In such case the grafted cylinder should have a diameter slightly smaller than that of the target site. It is noted that cartilage may be initially provided being too large, in which case it would be reshaped as needed (by removal excess tissue) before grafting.

Implantation of viable cartilage in accordance with the invention may be performed in any organ comprising cartilage, for example, ear, nose, or any articular joint, such as knee, elbow, shoulder, hip, etc.

As detailed above, the present invention provides frozen viable cartilage. Such cartilage may remain viable for an extended period of time (practically - indefinitely), and may thus be banked. Storage and banking may be done in any method known in the art provided that the temperature of the frozen viable cartilage would not be substantially increased in an uncontrolled manner and that it would not be prematurely thawed. Any cold chamber having a temperature that is -80°C or below, preferably -130°C and below (e.g. liquid nitrogen vapor) or even -196°C and below (such as liquid nitrogen) would suffice. This can allow useful or necessary tests to be performed before the tissue is transplanted (e.g. screening for infectious diseases). Banking may enable doctors to choose a graft from a larger reservoir of tissue and thus allow better donor-recipient matching (e.g. size, diameter, source and target site, shape).

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Fig. 1** is a graphical representation of the temperatures measured during freezing within a bone portion and a cartilage portion of an osteochondral graft while being frozen in accordance with an embodiment of this invention;
**Figs. 2(a)****-(b)** depict Haematoxilin and Eosine (H&E) staining of sheep cartilage. **Fig. 2(a)** shows fresh cartilage, whilst **Fig. 2(b)** shows cartilage that was frozen and thawed in accordance with an embodiment of the present invention.
**Figs. 3(a)****-(c)** are X-ray photographs of sheep knees before and after implantation of osteochondral cylinders. **Fig. 3(a)** depicts a healthy knee before surgery, **Fig. 3(b)** depicts a knee two months after surgery and **Fig. 3(c)** depicts a knee seven months after surgery.

### DETAILED DESCRIPTION OF THE INVENTION

In the following, all materials were purchased from Sigma St. Louis, USA unless specified otherwise.

### 1- freezing of osteochondral cylinders from sheep.

Fresh cadaver sheep legs were purchased from a slaughter house (Holon Slaughter house, Israel), and all manipulations of tissue samples were done in a sterile manner. Osteochondral cylinders, 12 mm in diameter, were drilled from sheep knee chondyle using a power surgery drill (Imex, Veterinary Inc. Texas, USA). Harvested cylinders were maintained in a buffered physiological solution containing 0.9% NaCl (Sigma, St. Louis, USA) and 3% antibiotics (Penicilin/Streptomycin/ Nystatin, Biological Industries, Beit Haemek, Israel) until completion of harvesting.

10 ml cryopreservation solution (comprising nutrient mixture F-12 (HAM), 1.78M Ethylene Glycol and 1% antibiotic (penicilin/streptomycin/nystatin)) was put in a conventional 16 mm (in diameter) glass tube. The harvested cylinders were inserted into the tubes with the cartilage part of the cylinder close to the tip of the tube. Cylinders were held at 4°C for a period of 30 minutes to 6 hours before freezing.

The cylinders were frozen using Multi Thermal Gradient (MTG) freezing apparatus (IMT, Israel) comprising 4 cooling blocks. Seeding was initiated by touching the leading end of each tube with cotton wool soaked with liquid nitrogen before insertion into the device. Immediately upon seeding, the cylinders were inserted into a first block and then moved through the blocks at 0.022mm/sec. The block temperatures, at their distal ends, were 0°C, -6°C, -46°C and -86°C. The cooling rates were 0.1°C/min from 0°C to -6°C and 0.4°C/min from -6°C to -86°C. After freezing the cylinders were transferred for storage in liquid nitrogen (LN) (temperature -130°C- -196°C).

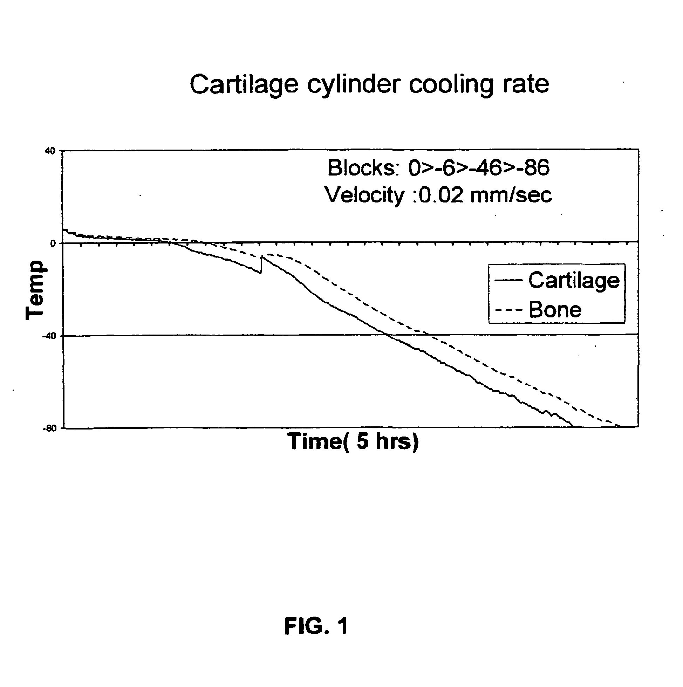

**Fig. 1** is a graph which depicts the temperature change within the cylinder during freezing, measured using two thermocouples (TCs) (ALMEMO^{R} 2290-4). One TC was inserted into bone tissue within the cylinder and the other TC was inserted within a cartilage portion of the cylinder. As seen, the cylinder was frozen in two different rates, the first rate being slower than the second rate. Between the two freezing rates there is a short isotherm, wherein the temperature is measured to rise rather than fall. This is due to the fact that crystallization, which occurs at the freezing point, is an exogenous reaction. In directional freezing, the heat so generated is transferred to the warmer portion of the sample (i.e. against the direction of the temperature gradient). Thus the rising of temperature does not substantially affect the art of freezing of the cylinder.

### 2- Thawing of osteochondral cylinders from sheep.

Tubes containing frozen articular cartilage cylinders (frozen according to the freezing protocol detailed in point 1 above) were removed from LN and were held at room temperature for 100 seconds (slow warming). They were then transferred to a 68°C water bath for 10 seconds and then to a 37°C water bath until cylinders were brought back to physiological temperatures (rapid warming).

The cryopreservation solution was washed by transferring the thawed cylinders through a series of solutions with decreasing concentrations of sucrose (0.5M, 0.25M and 0.125M). Sucrose (Sigma, St. Louis, USA) was dissolved in a solution composed of 89% F-12 HAM medium, 1% antibiotic (Penicilin/Streptomycin/Nystatin) and 10% FCS (all from Biological Industries Beit Haemek, Israel). After at least 5 minutes in each solution the thawed cylinders were kept in a F12 solution with 1% antibiotics Penicilin/Streptomycin/Nystatin-Biological Industries Beit Hamemek, Israel) and 10% FCS (Biological Industries Ltd.).

### 3- Survival of chondrocytes.

Different *in vitro* assays were used to assess the quality and viability of chondrocytes populating articular cartilage cylinders after freezing and thawing as described in points 1 and 2 above. The results of thawed cartilage cylinders were compared to those of fresh ones.

### Cartilage cultures

Thawed cartilage cylinders were obtained in accordance with point 2 above. Fresh cartilage cylinders were harvested along with the cylinders that were frozen but were assayed as follows, upon harvesting and without freezing. The cartilage from the cylinders was cut to cubes measuring ca. 2x2x1 mm and were assayed as follows.

### Cell viability assays

Table 1 summarizes the experimental results of experiments conducted to show the post-thaw viability of cartilage cells after being frozen and thawed in accordance with this invention. Fresh cartilage and samples that were frozen at different rates are compared.

**Table 1. In vitro sheep knee cartilage freezing results**

| | | | | |
|---|---|---|---|---|
| Cooling Rate (°C/min) | 0 (fresh cartilage) | 0.6 | 0.6 | 0.4 |
| Velocity (mm/sec) | 0 (fresh cartilage) | 0.1 | 0.05 | 0.025 |
| Cell culture^{*} | 100% | 40% | 40% | 80% |
| XTT* OD/mg tissue | 100% | 5% | 44% | 40% |
| LIVE/DEAD Viability (average ±sd); N=5 | 63.46±11/69 | 32.48±3.06 | 41.52±4.24 | 44.86±6.48 |
| Survival^{*} | 100% | 50% | 65% | 69% |

| | | | | |
|---|---|---|---|---|
| * the percentage was calculated in comparison to fresh cartilage | | | | |

The thawed or fresh tissue cubes (5 samples of each) were cultured in F12 (Biological Industries Ltd., Beit Haemek, Israel) supplemented with 10% FCS (Fetal Calf Serum Biological Industries Ltd., Beit Haemek, Israel).

The experimental protocols corresponding to Table 1 were as follows:

### 1. Cell culture assay

The samples were observed during incubation to confirm that chondrocytes migrated out of the cubes. Table 1 shows the proportion of cultures in which cartilage cells migrated out of the cubes within two weeks.

### 2. XTT-cell metabolism assay

The XTT reagent kit (Biological Industries Ltd., Beit Haemek, Israel) was used in accordance with the manufacturer's instructions. In this method the optical density (OD) is proportional to the number of living cells and their metabolic state.

Cartilage slices were incubated in a 96 well micro titer plates in complete culture medium (89% F12, 1% antibiotics, 10% FCS). A well containing slices of cartilage boiled for 2 minutes was used as control for non specific uptake by dead cells, and showed no significant difference in OD from the background. OD results were divided in cartilage wet weight to normalize for small differences in sample size.

### 3. Live/Dead Ratio

The LIVE/DEAD Viability Kit (L-7011 Molecular Probes, Oregon, USA), was used in accordance with the manufacturer's instructions. In this method live cells with intact membranes are observed as bright green, whereas cells with damaged membranes are observed red. Viability is expressed as number of green cell/total number of cells (green + red cells).

### 4. Post cryopreservation histology

For the histological studies of cartilage cryopreservation, fresh osteochondral cylinders and osteochondral cylinders (diameter 2mm) that were frozen and thawed as described above were fixated by immersion in 4% buffered formaldehyde . The fixated tissue was washed for 30 minutes under running tap water and then decalcified for 2 hours using Calci-Clear Rapid (National Diagnostics, Atlanta, USA). The cylinders were then washed again for 5 hours under running tap water. The samples were processed to a paraffin block using Tissue Tek VIP 5 (Sakura, Japan). Paraffin blocks were sectioned to 4µm sections and mounted on microscope slides.

Slides were stained using Haematoxilin and Eosin (Sigma, St. Louis, Usa) according to standard procedures ("Histological techniques and their diagnostic applications", J. D. Bancroft and H. C. Cook, Churchill-Livingstone, 1994).

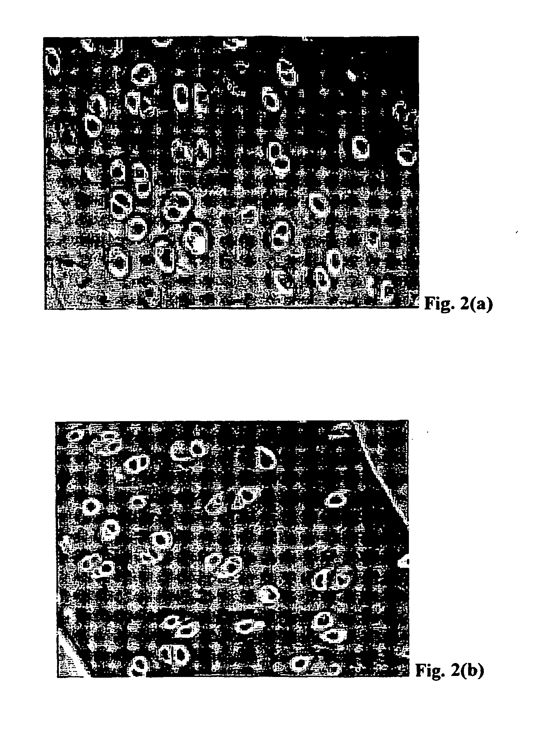

Examplary results are presented in **Figs. 2(a)-2(b)****,** showing that both tissues had similar chondrocyte density and cell viability. These results were reproducible and observed in all assayed cases.

### 4- Grafting of cryopreserved osteochondral grafts

The following examples encompassed 17 skeletally mature female Assaf sheep, nearly 1 year old. In each sheep two osteochondral cylinders were removed from the back-right knee. One of the osteochondral cylinders was autografted into the location from which the other cylinder was removed, and a thawed allograft was implanted in its original location.

Osteochondral cylinders for grafting were obtained from cadaver sheep legs which were frozen and thawed in accordance with the above protocols, with the following modifications (since the osteochondral cylinders were to be grafted into live sheep's knees). At the final stage of the thawing protocol, the 10% FCS was replaced with 10% serum that was prepared from the sheep into which the osteochondral cylinder was to be transplanted. Additionally, all manipulations of tissue samples were done in a sterile manner.

### 1. Grafting Procedure

Under general anesthesia, sheep lying supine, the right hind knee was prepared and draped, including shaving of the wool. Using a lateral para-patellar approach, a longitudinal incision of the skin and subcutaneous tissue was performed. A lateral arthrotomy was performed by extension of the incision through the para-patellar fascia, thereby exposing the patello-femoral joint. The patella was then medially everted in order to facilitate full exposure of both femoral condyles. The exposure was further enhanced by maximal knee flexion. Meticulous preservation of the common tendon of origin of the peroneus tertius and extensor digitorum longus muscles was performed; this tendon originates in the extra-articular lateral portion of the lateral femoral condyle and spans the medial intra-articular aspect of the lateral femoral condyle.

Using a drill, a 9.5mm diameter osteochondral cylinder was then removed from the central weight-bearing portion of the medial femoral condyle. The cylinder was placed in gauze soaked with normal saline (0.9% NaCl) for subsequent transplantation as an autograft into the lateral femoral condyle. The base of the defect formed was further deepened in order to match the length of the allograft to be transplanted; this correct sizing allows a smooth congruent articular surface. After copious irrigation with normal saline, the defect was filled using a press-fit technique, with the thawed cryopreserved allograft.

Similar drilling with a 6.5 mm drill was performed over the central weight bearing area of the lateral femoral condyle, taking care not to injure the medially placed common tendon of the peroneus tertius and extensor digitorum longus muscles. The cylinder removed from the medial femoral condyle was then similarly transplanted as an autograft, into the lateral femoral condyle.

After irrigation with normal saline and confirmation of haemostasis, the patella was reduced and the knee was placed through a full range of passive flexion and extension; this confirmed congruency and press-fit stability of the transplanted cylinders. The lateral para-patellar fascia was then sutured using an absorbable vicryl 2-0 suture (Johnson&Johnson); the subcutaneous tissue was similarly sutured with vicryl 2-0 suture. Marcaine (0.5% Bupivacaine HCl injection, Kamada, Beit-Kama, Israel) was injected into the knee joint for early post-operative analgesia. Staples were used for skin closure followed by a bandage which was stabilized by suture to the surrounding wool. The sheep was removed from the operating table and taken to the recovery area for extubation. After the operation, when the sheep were back on their feet, they were moved to their natural environment, the herd.

### 2. Grafting Results

### (a) visual assay

Within a very short time after the operation (as early as 5 days) the sheep were observed to be moving freely. Follow-up studies were carried out for a period of up to 1 year post surgery, and the sheep were scored visually for their walking ability.

The following scoring system was used: (1)-the leg is not being used, (2)-the leg is on the floor but there is no weight bearing, (3)-leg on the floor with weight bearing, (4)-slight limping, (5)-normal. Scores of the walking ability of sheep that underwent surgery during one month-span are shown in **Table 2** below. These are the average results provided by three independent observers. In total, 13 of 17 sheep have shown total recovery of knee function (both for walking and for standing).

**Table 2. Sheep walking scores after surgery**

| Sheep No. | Weeks post surgery | | | | | | | | | | | | | | | | | |
|---|---|---|---|---|---|---|---|---|---|---|---|---|---|---|---|---|---|---|
| | 2 | 3 | 4 | 5 | 6 | 7 | 9 | 10 | 11 | 12 | 13 | 14 | 16 | 17 | 18 | 20 | 21 | 22 |
| 3617 | - | - | 4 | - | - | 4 | - | - | 4 | - | - | 5 | - | - | 5 | - | - | 5 |
| 3527 | - | - | 3 | - | - | 5 | - | - | 5 | - | - | 5 | - | - | 5 | - | - | 5 |
| 3451 | - | - | 3 | - | - | 4 | - | - | 5 | - | - | 5 | - | - | 5 | - | - | 5 |
| 3616 | - | - | 3 | - | - | 5 | - | - | 5 | - | - | 5 | - | - | 5 | - | - | 5 |
| 3664 | - | 5 | - | - | 5 | - | - | 5 | - | - | 5 | - | - | 5 | - | - | 5 | - |
| 3651 | 3 | - | - | 4 | - | - | 5 | - | - | 5 | - | - | 5 | - | - | 5 | - | - |

### (b) X-ray photography

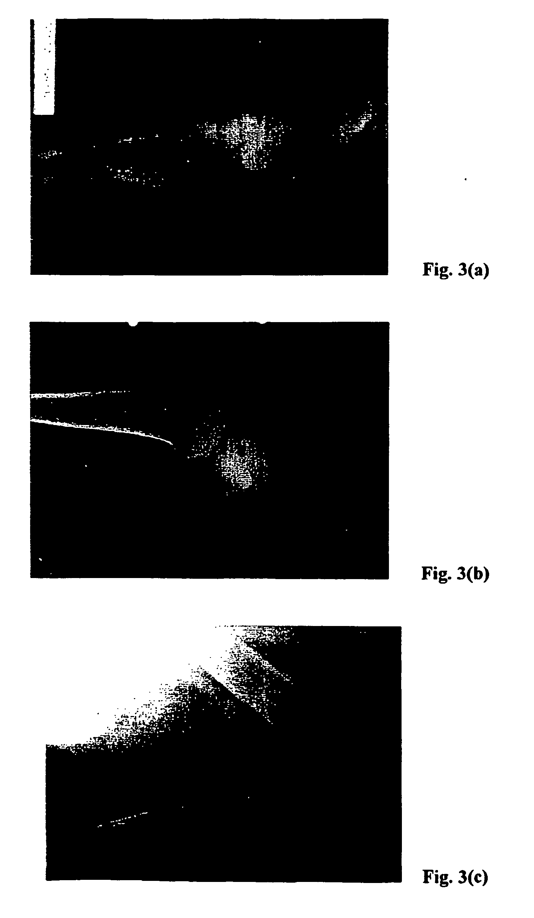

Sheep were X-ray photographed in order to estimate the degree of healing after the transplantation. Examples of such photographs are shown **in** Fig. **3(a)****-(c).** The X-rays of all sheep showed full incorporation of the graft in the subchondral level 2 months after transplantation. After 4 months there was no difference between an autograft and an allograft. After 7 months there was full incorporation of bone and cartilage with a smooth joint surface. No degenerative changes of the joint were observed. Incorporation of the graft was not affected in cases of patellar dislocation/sublaxation (4 out of 17). The x-ray results were also confirmed by magnetic resonance (MR) artrogram and computerized tomography (CT) artrogram, that was performed on the excised legs of a sacrificed sheep, 10 months after the implantation.

### (c) Post-mortem assays

In addition to the above, sheep were sacrificed about 10 month-year after transplantation, and the implants were assayed in all sacrificed sheep implants. Both allograft and autograft implants underwent complete fusion with the surrounding bone and cartilage. Slices of knee cartilage within and surrounding the implant were observed using the above detailed live/dead ratio assay. Viable chondrocytes were observed in both the surface of the implant and in its deepest level.

### 5-Alternative freeze thaw protocol

In an alternative experiment, osteochondral cylinders prepared for freezing as described (in point 1 above) were drilled in the bone part with a thin (2mm) drill and a screw with a looped end was inserted into the hole. A string about twice the length of the tube or longer was attached to the loop (although any length of string or wire that would allow pulling the cylinder out of the receptacle by hand or tool would do). The cylinders were frozen. In thawing, tubes at about -20°C were opened under a sterile hood and the still frozen cylinders were pulled out by force using the attached string. Ice surrounding the osteochondral cylinder was chipped off gently and the cylinders were plunged into a sterile swirling bath at 50°C for 10 seconds. Then the cylinders were transferred into clean receptacles with PBS at room temperature. The thawed cylinders were stained for viability using the LIVE/DEAD Viability Kit, as described above. As a control, cylinders were treated as described above, but without drilling. Accordingly, the control samples were thawed within the receptacles. The results of three experiments are summarized in Table 3. Viability is expressed in comparison with the viability of control cartilage taken from the same sheep.

**Table 3. Viability assay using different thawing protocols**

| **Experiment No.** | **% viability relative to control sample** |
|---|---|
| 1 | 225-250% |
| 2 | 150% |
| 3 | 200% |

### 6-General Remarks

### Harvesting and handling of Cartilage

In the above example, cartilage was obtained from sheep. However, the present invention may be carried out also in respect of tissue harvested from any other animal, including humans. After freezing and/or thawing the sample may be used for any purpose, including implantation in the donor or in a different recipient, whether or not of the same species, or for any other purpose such as research. The cartilage may be of any shape, size and diameter and can be taken from various places in the joint according to demand. It can be harvested in any desired manner. By this the donatated tissue is use to its full extent. To avoid contamination cartilage that is intended for implantation must be handled in a sterile manner, tissue that will not be frozen will be taken for pathogen screening.

For example, in sheep #3480 staining of unoperated knee showed 74% live cells whilst operated knee had 56%. In sheep #3435 76% cells were alive in the autograft (fresh) and 61% in the allograft (thawed). In sheep #3432 cartilage was thin but exhibited 100% live cells in both grafts.

Preferably, the frozen tissue is an osteochondral graft comprising articular cartilage removed from the femoral chondyls of the knee and may be in the form of a cylinder measuring 3mm to 50mm in diameter.

The harvested cartilage may be maintained for the duration of the harvesting procedure and for a short time prior to freezing under any conditions compatible with the tissue survival, and preferably in 0.9% NaCl solution containing 3% antibiotic solution that is microbially effective, while sparing the cells which are important for long-term tissue maintenance. The tissue is normally kept in room temperature until insertion into a cryoprotecting solution and then moved to 4°C, where it may be maintained for several hours before freezing.

### Cryopreservation

Any conventional buffered physiological solution can be used in practicing the present invention. For example - tissue culture media and simple buffered salt solutions may be used. A cryopreserving agent is added in solution to the osteochondral tissue to protect the cells during freezing, preferably ethylene glycol, although other suitable cell-penetrating organic solutes can be used, such as polyalcohols (for example, DMSO, ethylene glycol propylene glycol, glycerol and butane diol); and alkyl sulphoxides (for example, methyl ethyl sulphoxide, DMSO, diethylsulphoxide, dibutylsulphoxide, methylbutyl sulphoxide, and ethylbutylsulphoxide).

The cryopreservation solution may comprise for example 5 to 200ml of buffered physiological solution and a cell-penetrating organic solute in a concentration from about 0.5M to about 3M. Antibiotic solution of any kind may be included having the preferred concentration of 0.5% to 5%. The volume of solution used is such that the tissue would be completely immersed therein and can be easily determined by one skilled in the art, and is dependent upon the size of the tissue to be preserved.

Freezing can be done using any apparatus or method that will allow directional freezing of the cartilage, such as the Multi Thermal Gradient (MTG) freezing apparatus (IMT, Israel) that was used above. Block temperatures should impose on the viable cartilage a gradient beginning at a temperature above the freezing temperature of the solution, preferably between 5°C and 0°C, and ending at a temperature below the freezing temperature wherein recrystallization is practically non-existent. The cartilage may be cooled at any cooling rate that is sufficiently slow to prevent damage to the chondrocytes and preferably between 0.01°C/min and 3°C/min , or even 0.1°C/min and 3°C/min.

After freezing is completed the tubes may be stored in any cold storage facility at -130°C to -196°C. The samples may be kept practically indefinitely, and in any case may survive storage from 24 hours to 6 months before thawing.

### Thawing

It is appreciated by a person skilled in the art of the invention that according to the thawing method of the invention the viable cartilage is thawed in two stages. In the first stage the tissue may be thawed at any desired rate. However, it is preferably conducted at a slow warming rate (0.1°C/min-200°C/min) in order to avoid fracture of the tissue. In the second stage samples are brought to the melting point by bringing their temperature to 0°C at rapid warming rate (50°C/min-1000°C/min) in order to avoid recrystalization which is known to damage biological entities.

After thawing, the cryoprotectant is removed as follows: The viable cartilage thawed as described above is placed using a sterile technique in a solution containing biocompatible sugar, a serum of any species and antibiotics in a buffered physiological solution for 5 to 10 minutes. Any non-cell membrane permeable biocompatible sugar, polyol or other organic solute can be used, such as sucrose, mannitol, sorbitol, trehalose, fructose, glucose, raffinose, maltose, xylitol, amino acids or the like.

The dilution of the cryoprotectant concentration by the biocompatible sugar solution is preferably in decreasing steps of at least half the molarity of the previous step. Thus, if the original cryoprotectant concentration is 2M, the first dilution step would employ 1M sugar.

## Claims

1. A method for the generation of frozen viable articular cartilage tissue, said method comprising:
**(a)** providing a receptacle containing viable articular cartilage tissue in a cryopreservation solution at a temperature above the freezing temperature of the cryopreservation solution;
**(b)** directionally cooling said viable articular cartilage tissue to a temperature below said freezing temperature at a cooling rate of 0.01°C/min to 3°C/min, whereby the receptacle is moved along one or more consecutive temperature gradients ranging from a temperature above said freezing temperature to a temperature below said freezing temperature, thereby generating directionally frozen viable articular cartilage tissue.

2. The method of Claim 1, wherein in step (b) the velocity of movement along the at least one temperature gradient in step (b) is between 0.002 mm/sec and 5 mm/sec.

3. The method of any one of Claims 1 to 2, wherein at least one of the one or more consecutive temperature gradients in step (b) is between 0.1°C/mm to 50°C/mm.

4. The method of anyone of Claims 1 to 3, comprising after step (b):
(c) transferring the receptacle to storage at a temperature below the freezing point of said cryopreservation solution.

5. The method of any one of Claims 1 to 4, wherein the viable articular cartilage tissue is human articular cartilage.

6. The method of any one of Claims 1 to 5, wherein the viable articular cartilage tissue comprises osteochondral tissue.

7. The method of Claim 6, wherein the osteochondral tissue is a osteochondral cylinder.

8. The method of any one of Claims 1 to 5, wherein the osteochondral cylinder is taken from a joint.

9. The method of any one of Claims 1 to 8, comprising storing the frozen viable articular cartilage tissue at a temperature between -130°C to -196°C for at least 24 hours.

10. The method of Claim 9, comprising storing said frozen viable articular cartilage tissue for 6 months.

11. A method for thawing frozen viable articular cartilage tissue that was frozen in a solution, the method comprising:
**(a)** providing a receptacle containing frozen viable articular cartilage tissue at an initial temperature below the glass transition temperature of the solution;
**(b)** warming said viable articular cartilage tissue from said initial temperature to an intermediate temperature being at least about said glass transition temperature or above said glass transition temperature but no more than the temperature wherein recrystallization would begin to occur at any point in the cartilage;
**(c)** warming said viable articular cartilage tissue from said intermediate temperature to a temperature that is at least substantially equal to the melting temperature of the solution, by removing said viable articular cartilage tissue from said receptacle and contacting the said viable articular cartilage tissue with an environment having a temperature of 0°C or more; said warming being at a rate sufficiently high to minimize recrystallization; thereby obtaining thawed viable articular cartilage tissue
wherein said viable articular cartilage tissue is connected to a pulling member and the removing of said viable articular cartilage tissue from said receptacle is executed by pulling on said pulling member.

12. The method of Claim 11, wherein said warming in step (b) is at a rate of between 0.1°C/min and 200°C/min.

13. The method of anyone of Claim 11, wherein said warming in step (c) is at a rate of between 50°C/min and 1000°C/min.

14. The method of any one of Claims 11 to 13, wherein said intermediate temperature is less than -10°C.

15. The method of Claim 14, wherein said intermediate temperature is in a range selected of -20 to -80°C.

16. The method of any one of Claims 11 to 15, wherein said pulling member is attached to the bone portion of said viable articular cartilage tissue via a screw.

## Patentansprüche

1. Verfahren zur Erzeugung von gefrorenem, lebensfähigem Gelenkknorpelgewebe, wobei das Verfahren umfasst:
(a) Bereitstellen eines Behälters, der lebensfähiges Gelenkknorpelgewebe in einer Kryokonservierungslösung bei einer Temperatur oberhalb der Gefriertemperatur der Kryokonservierungslösung enthält;
(b) gerichtetes Kühlen des lebensfähigen Gelenkknorpelgewebes auf eine Temperatur unter der Gefriertemperatur mit einer Kühlrate von 0,01°C/min bis 3°C/min, wobei der Behälter entlang eines Temperaturgradienten oder mehrerer aufeinander folgender Temperaturgradienten, reichend von einer Temperatur über der Gefriertemperatur zu einer Temperatur unter der Gefriertemperatur, bewegt wird, wodurch ein gerichtet gefrorenes lebensfähiges Gelenkknorpelgewebe erzeugt wird.

2. Verfahren nach Anspruch 1, wobei in Schritt (b) die Geschwindigkeit der Bewegung entlang des wenigstens einen Temperaturgradienten in Schritt (b) zwischen 0,002 mm/s und 5 mm/s liegt.

3. Verfahren nach einem der Ansprüche 1 bis 2, wobei wenigstens einer des einen Temperaturgradienten oder der mehreren aufeinander folgenden Temperaturgradienten in Schritt (b) zwischen 0,1°C/mm bis 50°C/mm ist.

4. Verfahren nach einem der Ansprüche 1 bis 3, umfassend nach Schritt (b) :
(c) Transferieren des Behälters zu einer Lagerung bei einer Temperatur unter dem Gefrierpunkt der Kryokonservierungslösung.

5. Verfahren nach einem der Ansprüche 1 bis 4, wobei das lebensfähige Gelenkknorpelgewebe humaner Gelenkknorpel ist.

6. Verfahren nach einem der Ansprüche 1 bis 5, wobei das lebensfähige Gelenkknorpelgewebe osteochondrales Gewebe umfasst.

7. Verfahren nach Anspruch 6, wobei das osteochondrale Gewebe ein osteochondraler Zylinder ist.

8. Verfahren nach einem der Ansprüche 1 bis 5, wobei der osteochondrale Zylinder aus einem Gelenk entnommen wurde.

9. Verfahren nach einem der Ansprüche 1 bis 8, das ein Lagern des gefrorenen lebensfähigen Gelenkknorpelgewebes bei einer Temperatur zwischen -130°C und -196°C für wenigstens 24 Stunden umfasst.

10. Verfahren nach Anspruch 9, das Lagern des gefrorenen lebensfähigen Gelenkknorpelgewebes für 6 Monate umfasst.

11. Verfahren zum Auftauen von gefrorenem lebensfähigem Gelenkknorpelgewebe, das in einer Lösung gefroren war, wobei das Verfahren umfasst:
(a) Bereitstellen eines Behälters, der gefrorenes lebensfähiges Gelenkknorpelgewebe mit einer Anfangstemperatur unter der Glasübergangstemperatur der Lösung enthält;
(b) Erwärmen des lebensfähigen Gelenkknorpelgewebes ab der Anfangstemperatur zu einer Zwischentemperatur, die wenigstens etwa die Glasübergangstemperatur ist oder über der Glasübergangstemperatur liegt, aber nicht höher als die Temperatur ist, bei der an einem beliebigen Punkt im Knorpel eine Rekristallisation beginnen würde;
(c) Erwärmen des lebensfähigen Gelenkknorpelgewebes von der Zwischentemperatur auf eine Temperatur, die wenigstens im Wesentlichen gleich der Schmelztemperatur der Lösung ist, indem das lebensfähigen Gelenkknorpelgewebe aus dem Behälter entfernt wird und das lebensfähige Gelenkknorpelgewebe mit einer Umgebung, die eine Temperatur von 0°C oder mehr hat, in Kontakt gebracht wird; wobei das Erwärmen mit einer Rate erfolgt, die ausreichend ist, um eine Rekristallisation zu minimieren, wodurch aufgetautes lebensfähiges Gelenkknorpelgewebe erhalten wird,
wobei das lebensfähige Gelenkknorpelgewebe mit einem Zugelement verbunden wird und die Entfernung des lebensfähigen Gelenkknorpelgewebes aus dem Behälter durch Ziehen an dem Zugelement durchgeführt wird.

12. Verfahren nach Anspruch 11, wobei das Erwärmen in Schritt (b) mit einer Rate von zwischen 0,1°C/min und 200°C/min erfolgt.

13. Verfahren nach Anspruch 11, wobei das Erwärmen in Schritt (c) mit einer Rate von zwischen 50°C/min und 1000°C/min erfolgt.

14. Verfahren nach einem der Ansprüche 11 bis 13, wobei die Zwischentemperatur weniger als -10°C ist.

15. Verfahren nach Anspruch 14, wobei die Zwischentemperatur in einem Bereich, der von -20 bis -80°C liegt, ausgewählt ist.

16. Verfahren nach einem der Ansprüche 11 bis 15, wobei das Zugelement durch eine Schraube an dem Knochenteil des lebensfähigen Gelenkknorpelgewebes befestigt ist.

## Revendications

1. Procédé pour générer du tissu cartilagineux viable congelé, ledit procédé comprenant :
(a) la fourniture d'un récipient contenant le tissu cartilagineux articulaire viable dans une solution de cryoconservation à une température supérieure à la température de congélation de la solution de cryoconservation ;
(b) le refroidissement de manière directionnelle dudit tissu cartilagineux articulaire viable à une température inférieure à ladite température de congélation à une vitesse de refroidissement allant de 0,01 °C/minute à 3 °C/minute, dans lequel le récipient est déplacé le long d'un ou plusieurs gradients de température consécutifs allant d'une température supérieure à ladite température de congélation à une température inférieure à ladite température de congélation, générant ainsi du tissu cartilagineux articulaire viable congelé de manière directionnelle.

2. Procédé selon la revendication 1, dans lequel dans l'étape (b), la vitesse de mouvement le long d'au moins un gradient de température dans l'étape (b) est comprise entre 0,002 mm/seconde et 5 mm/seconde.

3. Procédé selon l'une quelconque des revendications 1 à 2, dans lequel au moins un d'un ou plusieurs gradients de température consécutifs dans l'étape (b) est compris entre 0,1 °C/mm et 50 °C/mm.

4. Procédé selon l'une quelconque des revendications 1 à 3, comprenant après l'étape (b) :
(c) le transfert du récipient vers un stockage à une température inférieure au point de congélation de ladite solution de cryoconservation.

5. Procédé selon l'une quelconque des revendications 1 à 4, dans lequel le tissu cartilagineux articulaire viable est un cartilage articulaire humain.

6. Procédé selon l'une quelconque des revendications 1 à 5, dans lequel le tissu cartilagineux articulaire viable comprend le tissu ostéochondral.

7. Procédé selon la revendication 6, dans lequel le tissu ostéochondral est un cylindre ostéochondral.

8. Procédé selon l'une quelconque des revendications 1 à 5, dans lequel le cylindre ostéochondral est prélevé à partir d'une articulation.

9. Procédé selon l'une quelconque des revendications 1 à 8, comprenant le stockage du tissu cartilagineux articulaire viable congelé à une température comprise entre -130 °C et -196 °C pendant au moins 24 heures.

10. Procédé selon la revendication 9, comprenant le stockage dudit tissu cartilagineux articulaire viable congelé pendant 6 mois.

11. Procédé de décongélation de tissu cartilagineux articulaire viable congelé qui est congelé dans une solution, le procédé comprenant :
(a) la fourniture d'un récipient contenant un tissu cartilagineux articulaire viable congelé à une température initiale inférieure à la température de transition vitreuse de la solution ;
(b) le réchauffement dudit tissu cartilagineux articulaire viable de ladite température initiale à une température intermédiaire étant au moins à peu près celle de ladite température de transition vitreuse ou supérieure à ladite température de transition vitreuse, mais pas supérieure à la température à laquelle une recristallisation pourrait commencer à se produire à n'importe quel point dans le cartilage ;
(c) le réchauffement dudit tissu cartilagineux articulaire viable de ladite température intermédiaire à une température qui est au moins sensiblement égale à la température de fusion de la solution, en retirant ledit tissu cartilagineux articulaire viable dudit récipient et en mettant en contact ledit tissu cartilagineux articulaire viable avec un environnement ayant une température de 0 °C ou plus ;
ledit réchauffement étant réalisé à une vitesse suffisamment élevée pour minimiser la recristallisation ; obtenant ainsi du tissu cartilagineux articulaire viable décongelé,
dans lequel ledit tissu cartilagineux articulaire viable est relié à un élément de traction et le retrait dudit tissu cartilagineux articulaire viable dudit récipient est exécuté en tirant sur ledit élément de traction.

12. Procédé selon la revendication 11, dans lequel ledit réchauffement dans l'étape (b) est réalisé à une vitesse comprise entre 0,1 °C/minute et 200 °C/minute.

13. Procédé selon la revendication 11, dans lequel ledit réchauffement dans l'étape (c) est réalisé à une vitesse comprise entre 50 °C/minute et 1000 °C/minute.

14. Procédé selon l'une quelconque des revendications 11 à 13, dans lequel ladite température intermédiaire est inférieure à -10 °C.

15. Procédé selon la revendication 14, dans lequel ladite température intermédiaire est comprise dans la plage allant de -20 à -80 °C.

16. Procédé selon l'une quelconque des revendications 11 à 15, dans lequel ledit élément de traction est attaché à la partie osseuse dudit tissu cartilagineux articulaire viable au moyen d'une vis.
